# EUROPEAN PATENT APPLICATION

(11) **EP 4 803 609 A1**
(43) Date of publication of application: **09.09.2026**
(21) Application number: 24886401.9
(22) Date of filing: 04.11.2024
(51) Int. Cl.: C12N 1/20, C12N 1/38, A23L 2/38, A23L 33/135, C12R 1/25

(54) **LACTIC ACID BACTERIA FERMENTED BEVERAGE USING ULTRAFINE RED PEPPER POWDER, AND PREPARATION METHOD THEREFOR**

(30) Priority: 02.11.2023 KR 20230150101
(71) Applicant: CJ Cheiljedang Corporation, Seoul 04560 (KR)
(72) Inventor: SHIN, Dongjoo, Seoul 04560 (KR); JUNG, Hee Kyoung, Seoul 04560 (KR); HONG, Na Youn, Seoul 04560 (KR); WOO, A Mi, Seoul 04560 (KR); SEOW, YI Xin, Seoul 04560 (KR)
(74) Representative: Meissner Bolte Partnerschaft mbB
(86) International application number: PCT/KR2024/017173
(87) International publication number: WO 2025/095731

(57) **Abstract**

The present disclosure relates to: a medium composition for culturing lactic acid bacteria, comprising ultra-fine red pepper powder as an active ingredient; a method for culturing lactic acid bacteria using the medium composition for culturing lactic acid bacteria; a method for increasing the viable cell count of lactic acid bacteria; a lactic acid bacteria fermentation broth prepared according to the method for culturing lactic acid bacteria; a probiotic food composition comprising the lactic acid bacteria fermentation broth; and a probiotic beverage composition comprising the lactic acid bacteria fermentation broth.

## Description

### [Technical Field]

The present disclosure relates to: a medium composition for culturing lactic acid bacteria; a method for culturing lactic acid bacteria; a method for increasing the viable cell count of lactic acid bacteria; a lactic acid bacteria fermentation broth prepared according to the method for culturing lactic acid bacteria; a probiotic food composition comprising the lactic acid bacteria fermentation broth; and a probiotic beverage composition comprising the lactic acid bacteria fermentation broth.

### [Background Art]

Lactic acid bacteria, also referred to as lactic acid-producing bacteria, are important bacteria that inhabit the intestinal tract of mammals and inhibit abnormal fermentation caused by various microorganisms, and are therefore used as intestinal regulators. For example, *L. bulgaricus*, which is one of the earliest known lactic acid bacteria, is used in the production of yogurt and as a starter in the production of cheese or fermented butter. In addition, *L. acidophilus* are present in the intestines of humans and all mammals as well as other animals, and are used in the production of butter or milk and in the treatment of intestinal autointoxication. *L. lactis* produces DL-lactic acid, and since it is always present in milk, it is used in the production of butter or cheese. Thus, it is one of the most important lactic acid bacteria for dairy use.

Lactic acid bacteria can survive in the intestinal tract after ingestion, thereby exerting beneficial effects on the human body through various physiological activities, including activation of intestinal peristalsis by colonizing the intestine, inhibition of harmful microorganisms, promotion of immune-enhancing substances including vitamins, and alleviation of atopic skin conditions. Typically, a method for providing lactic acid bacteria as an ingestible food includes fermented milk produced by inoculating lactic acid bacteria into sterilized milk. In addition, lactic acid bacteria are contained in various fermented foods in which microorganisms can proliferate through fermentation processes, such as kimchi, cheonggukjang, and natto, which are well-known examples.

However, it is difficult to ingest a sufficient number of lactic acid bacteria through general fermented foods, and the ease of consumption from a single food is poor. Additionally, fermented milk products may cause allergies due to proteins derived from milk. To address these problems, research on methods of ingesting lactic acid bacteria through fermentation of plant-based (vegan) raw materials, such as coconut, has been conducted (Japanese Patent Application Publication No. 2014-233261). However, the development of plant-based fermented lactic acid bacteria products that exhibit excellent culturing efficiency while also having improved drinkability remains insufficient to date.

### [Disclosure]

### [Technical Problem]

The problem to be solved by the present disclosure is to provide a medium composition for culturing lactic acid bacteria, a method for culturing lactic acid bacteria, a method for increasing the viable cell count of lactic acid bacteria, a lactic acid bacteria fermentation broth, and a probiotic beverage composition.

### [Technical Solution]

An object of the present disclosure is to provide a medium composition for culturing lactic acid bacteria, comprising ultra-fine red pepper powder having an average particle size of 10 µm to 50 µm as an active ingredient.

Another object of the present disclosure is to provide a method for culturing lactic acid bacteria, comprising a culturing step of inoculating and culturing lactic acid bacteria in the medium composition for culturing lactic acid bacteria.

Still another object of the present disclosure is to provide a method for increasing the viable cell count of lactic acid bacteria, comprising a culturing step of inoculating and culturing the lactic acid bacteria in the medium composition for culturing lactic acid bacteria.

Still another object of the present disclosure is to provide a lactic acid bacteria fermentation broth, prepared by the method for culturing lactic acid bacteria.

Still another object of the present disclosure is to provide a probiotic beverage composition, comprising the lactic acid bacteria fermentation broth as an active ingredient.

Still another object of the present disclosure is to provide a probiotic food composition, comprising the lactic acid bacteria fermentation broth as an active ingredient.

### [Advantageous Effects]

The medium composition for culturing lactic acid bacteria of the present disclosure, containing ultra-fine red pepper powder, and the method for culturing lactic acid bacteria using the same can increase fermentation efficiency of the lactic acid bacteria and improve the growth environment, thereby providing a lactic acid bacteria fermentation broth having an increased survival rate and increased viable cell count of the lactic acid bacteria.

In addition, the lactic acid bacteria fermentation broth produced using the medium composition and the method for culturing lactic acid bacteria of the present disclosure exhibits minimized occurrence of sedimentation, and thus can provide a probiotic beverage or food composition that has a high lactic acid bacteria content, improved drinkability, and improved storage stability, and is easy to ingest, thereby providing high applicability.

### [Brief Description of the Drawings]

FIG. 1 is a graph illustrating a particle size distribution according to results of particle size analysis of ultra-fine red pepper powder in Example 1.
FIG. 2 is a schematic diagram illustrating a process for preparing a lactic acid bacteria fermentation broth according to Examples 3-1 and 3-2.
FIG. 3 is a schematic diagram illustrating a method for preparing a probiotic beverage composition according to an embodiment of the present disclosure.
FIGS. 4 to 6 are graphs illustrating growth curves in media for strains CJLP133, CJLP243, and CJLP55, respectively. Original represents a growth curve of the medium of Comparative Example 1, and Ultra-fine represents a growth curve of the medium of Experimental Example 1 of the present disclosure using ultra-fine red pepper powder.
FIG. 7 is an image illustrating appearances of fermentation broths after culturing in the media of Comparative Example 1 and Experimental Example 1, and forms of respective red pepper powder samples used for culturing.

### [Detailed Description of Preferred Embodiments]

The present disclosure will be described in detail as follows. Meanwhile, each description and embodiment described herein can be applied to other descriptions and embodiments, respectively. That is, all combinations of various elements described herein fall within the scope of the present disclosure. Further, the scope of the present disclosure is not limited by the specific description described below. In addition, throughout the present specification, numerous papers and patent documents are referenced, and citations thereof are indicated. The content of the cited papers and patent documents is incorporated herein by reference in its entirety to describe the level of the technical field to which the present disclosure belongs and the contents of the present disclosure more clearly.

In view of the foregoing background, the present inventors have confirmed that containing ultra-fine red pepper powder having ultra-fine particles in a medium composition for culturing lactic acid bacteria can not only improve the growth environment of lactic acid bacteria and significantly increase their survival rate during fermentation, but also minimize the occurrence of sedimentation during fermentation, thereby enhancing storage stability and ease of ingestion, and thus have completed the present invention.

Hereinafter, the present disclosure will be described in more detail.

An aspect of the present disclosure provides a medium composition for culturing lactic acid bacteria, comprising ultra-fine red pepper powder as an active ingredient.

As used herein, the term "ultra-fine red pepper powder" refers to red pepper powder prepared by ultra-fine pulverization of red pepper, wherein the red pepper powder has a small particle size on an ultra-fine scale.

The average particle diameter of ultra-fine red pepper powder may be 10 µm to 50 µm, more specifically 15 µm to 45 µm, 20 µm to 45 µm, 23 µm to 45 µm, 25 µm to 45 µm, 15 µm to 40 µm, 20 µm to 40 µm, 23 µm to 40 µm, 25 µm to 40 µm, 15 µm to 35 µm, 20 µm to 35 µm, 23 µm to 35 µm, 25 µm to 35 µm, 15 µm to 30 µm, 18 µm to 30 µm, 20 µm to 30 µm, 23 µm to 32 µm, 25 µm to 35 µm, 22 µm to 28 µm, 23 µm to 30 µm, 23 µm to 28 µm, or 24 µm to 26 µm. When the ultra-fine red pepper powder has an average particle diameter within the above range, the growth environment of lactic acid bacteria can be improved, and fermentation efficiency can be increased.

In one example, the average particle diameter of ultra-fine red pepper powder may be a range defined by a lower boundary selected from 17 µm, 17.5 µm, 18 µm, 18.5 µm, 19 µm, 19.5 µm, 20 µm, 20.5 µm, 21 µm, 21.5 µm, 22 µm, 22.5 µm, 23 µm, 23.5 µm, 24 µm, 24.5 µm, 25 µm, 25.5 µm, 26 µm, 26.5 µm, and 27 µm, and/or an upper boundary selected from 27.5 µm, 28 µm, 28.5 µm, 29 µm, 29.5 µm, 30 µm, 30.5 µm, 31 µm, 31.5 µm, 32 µm, 32.5 µm, 33 µm, 33.5 µm, 34 µm, 34.5 µm, 35 µm, 35.5 µm, 36 µm, 36.5 µm, and 37 µm.

In one example, the ultra-fine red pepper powder may have a particle size distribution in which D10, D50, and D90 are all 100 µm or less. In one more specific example, the ultra-fine red pepper powder may have a particle size distribution in which D10, D50, and D90 are all 90 µm or less, 80 µm or less, 75 µm or less, or 70 µm or less.

As used herein, the term "particle size" refers to the size of particles, and, unlike particle diameter, which generally represents the size of particles in terms of diameter, the term "particle size" as used herein includes indirect indicators such as specific surface area. For a perfectly spherical particle, a simple relationship is established between the particle diameter and other measures of particle size. However, it is generally difficult to define the particle size by a single parameter, and it is therefore expressed as an average value of a representative length, such as an average diameter (an average value of lengths measured in two or more directions) or an equivalent diameter (a representative length obtained by assuming a polyhedral particle to have a simple geometric shape).

The particle size distribution can be measured by determining the minimum size, maximum size, average value, and the like of particles in a sample using a particle size analyzer. However, since the particle size distribution of a sample cannot be accurately determined based solely on the average particle size, particle size values corresponding to 10%, 50%, and 90% in a cumulative particle size distribution are expressed as D10, D50, and D90, respectively. The particle size distribution of the sample can be accurately represented by defining the particle size distribution curve based on these values. The particle size distribution may be measured by methods commonly used in the art, and is not limited to a particular method.

In one example, the particle size distribution of the ultra-fine red pepper powder of the present disclosure may be one in which D10 is 0.5 µm to 20 µm, 0.5 µm to 15 µm, 1 µm to 15 µm, 3 µm to 15 µm, 5 µm to 15 µm, or 8 µm to 12 µm; D50 is 5 µm to 40 µm, 5 µm to 35 µm, 15 µm to 30 µm, 10 µm to 35 µm, 20 µm to 30 µm, or 23 µm to 28 µm; and D90 is 20 µm to 100 µm, 20 µm to 80 µm, 20 µm to 60 µm, 30 µm to 70 µm, 40 µm to 70 µm, 30 µm to 60 µm, 40 µm to 60 µm, or 45 µm to 55 µm, but is not limited thereto.

Specifically, the ultra-fine red pepper powder may be comprised in an amount of 1 wt% to 20 wt% based on the total weight of the medium composition. More specifically, the ultra-fine red pepper powder may be comprised in an amount of 3 wt% to 17 wt%, more specifically 5 wt% to 15 wt%, and in one example, 6 wt% to 12 wt%, based on the total weight of the medium composition, but is not limited thereto. When the ultra-fine red pepper powder is comprised in the medium composition in an amount within the above range, sufficient nutrients can be provided without inhibiting the growth environment of lactic acid bacteria due to excessive powderization of the medium.

In the present disclosure, the medium composition for culturing lactic acid bacteria may further comprise fructose.

As used herein, the term "fructose" may also be referred to as levulose, and is one of the most important hexoses. It has a molecular formula of C₆H₁₂O₆, is colorless and hygroscopic, and has a melting point of 103°C to 105°C. Fructose is widely present in the plant kingdom, and is present in fruits either in a free form together with glucose or in sucrose, in which it is bound to glucose.

Specifically, the fructose may be comprised in an amount of 0.01 wt% to 10 wt% based on the total weight of the medium composition. More specifically, the ultra-fine red pepper powder may be comprised in an amount of 0.01 wt% to 5 wt%, more specifically 0.5 wt% to 5 wt%, and in one example, 1 wt% to 3 wt%, based on the total weight of the medium composition, but is not limited thereto. When fructose is comprised in the medium composition in an amount within the above range, fructose is provided as a nutrient for lactic acid bacteria in a lactic acid bacteria fermentation broth prepared using the medium composition, thereby increasing the shelf life of the prepared fermentation broth, and in particular improving the shelf life during refrigerated storage.

The medium composition for culturing lactic acid bacteria of the present disclosure may further comprise a buffer solution.

As used herein, the term "buffer solution" refers to a substance that functions to reduce changes in pH when an acidic or alkaline substance is added. For the purposes of the present disclosure, any buffer solution may be used without limitation, as long as it is capable of performing the role of preventing a rapid decrease in pH caused by fermentation of lactic acid bacteria in the medium composition for culturing lactic acid bacteria.

Specifically, the buffer solution may be comprised in an amount of 0.1 wt% to 15 wt% based on the total weight of the medium composition.

Specifically, in the present disclosure, the buffer solution may further comprise at least one of citric acid and trisodium citrate.

As used herein, the term "citric acid" is also referred to as citric acid, and its name is derived from the fact that it is particularly abundant in citrus fruits. Citric acid has a chemical formula of C₆H₈O₇ and is readily soluble in water, ethanol, and the like. It is used as a food additive for various purposes or as a preservative in therapeutic agents.

As used herein, the term "trisodium citrate" is also referred to as sodium citrate tribasic, and is a colorless transparent crystal or a white crystalline powder having a chemical formula of C₆H₅Na₃O₇·nH₂O (wherein n = 0 or 2). An aqueous solution thereof is slightly alkaline and is used as a pH regulator for foods and the like, an anti-rancidity agent for dairy products, a thickening agent for processed cheese, and an emulsifying and stabilizing agent for sherbet, ice cream, and the like.

Specifically, the citric acid may be comprised in an amount of 0.001 wt% to 5 wt% based on the total weight of the medium composition. More specifically, the citric acid may be comprised in an amount of 0.01 wt% to 3 wt%, more specifically 0.1 wt% to 3 wt%, and in one example, 0.5 wt% to 1 wt%, based on the total weight of the medium composition, and although the content range is not limited thereto, a buffer solution optimized for increasing the growth of lactic acid bacteria can be provided in the above content range.

Specifically, the trisodium citrate may be comprised in an amount of 0.1 wt% to 10 wt% based on the total weight of the medium composition. More specifically, the trisodium citrate may be comprised in an amount of 1 wt% to 10 wt%, more specifically 1 wt% to 6 wt%, and in one example, 1 wt% to 4 wt%, based on the total weight of the medium composition, and although the content range is not limited thereto, a buffer solution optimized for increasing the growth of lactic acid bacteria can be provided in the above content range.

Specifically, the medium composition for culturing lactic acid bacteria of the present disclosure may further comprise a remaining amount of purified water. Specifically, the purified water may be comprised in an amount of 50 wt% to 95 wt%, more specifically 70 wt% to 95 wt%, and in one example, 80 wt% to 90 wt%, based on the total weight of the medium composition, but is not limited thereto.

As used herein, the term "lactic acid bacteria" is a collective term referring to bacteria that obtain energy by fermenting sugars and produce a large amount of lactic acid, but is not particularly limited thereto, and the lactic acid bacteria may comprise at least one selected from the group consisting of *Lactobacillus* sp., *Bifidobacterium* sp., *Streptococcus* sp., *Lactococcus* sp., *Enterococcus* sp., *Pediococcus* sp., *Leuconostoc* sp., and *Weissella* sp., but are not limited thereto.

Specifically, the lactic acid bacteria may comprise at least one selected from the group consisting of *Lactobacillus plantarum*, *Lactobacillus casei*, *Lactobacillus rhamnosus*, *Lactobacillus acidophilus*, *Bifidobacterium bifidum*, *Bifidobacterium longum*, *Bifidobacterium breve*, *Streptococcus faecalis*, and *Lactococcus lactis subsp. lactis*, but are not limited thereto.

More specifically, the lactic acid bacteria may comprise at least one selected from the group consisting of *Lactobacillus plantarum* CJLP133, *Lactobacillus plantarum* CJLP243, and *Lactobacillus plantarum* CJLP55, but are not limited thereto.

The strain has been deposited with the Gene Bank of the Korea Research Institute of Bioscience and Biotechnology, and is a strain that can be readily obtained by those skilled in the art from the Gene Bank of the Korea Research Institute of Bioscience and Biotechnology.

As used herein, "culturing" refers to growing the lactic acid bacteria under properly controlled environmental conditions. The culturing process of the present disclosure may be performed in a suitable medium known in the art under suitable culturing conditions known in the art. Such a culturing process may be readily adjusted and used by those skilled in the art according to the selected strain. Specifically, the culturing may be batch culturing, continuous culturing, or fed-batch culturing, but is not limited thereto.

As used herein, the term "medium" refers to a mixed substance containing nutrients required for culturing lactic acid bacteria as a main component, and the medium supplies nutrients, growth factors, *etc*., including water, which are indispensable for survival and development. For the purposes of the present disclosure, the medium may contain ultra-fine red pepper powder as a main nutrient, and may further comprise fructose.

In addition, the medium composition for culturing lactic acid bacteria of the present disclosure may further comprise other additives required for culturing lactic acid bacteria, and may further comprise, for example, other carbon sources, nitrogen sources, phosphorus sources, inorganic compounds, amino acids, and/or vitamins.

Another aspect of the present disclosure provides a method for culturing lactic acid bacteria, comprising a culturing step of inoculating and culturing the lactic acid bacteria in a medium composition for culturing lactic acid bacteria comprising ultra-fine red pepper powder as an active ingredient.

The method for culturing lactic acid bacteria may further comprise a seed culturing step of obtaining a seed culture broth by culturing the lactic acid bacteria in a seed medium before the culturing step.

In the seed culturing step, any seed medium may be used as long as the medium is conventionally used for culturing lactic acid bacteria, and may be, for example, an MRS medium.

In the seed culturing step, the temperature of the seed medium may be 20°C to 50°C, or specifically 35°C to 40°C, but is not limited thereto.

In the seed culturing step, the seed culturing may be continued until a desired amount of seed culture broth is obtained, and may specifically be performed for 10 to 50 hours, but is not limited thereto.

Next, the culturing step refers to a step of inoculating lactic acid bacteria into the medium composition for culturing lactic acid bacteria comprising ultra-fine red pepper powder as an active ingredient, and culturing the lactic acid bacteria to carry out fermentation.

In particular, the medium composition for culturing lactic acid bacteria is defined in the same manner as described above.

The method for culturing lactic acid bacteria of the present disclosure is characterized in that, through lactic acid bacteria fermentation using ultra-fine red pepper powder as described above, it is possible to provide a lactic acid bacteria fermentation broth having an increased survival rate of lactic acid bacteria while exhibiting a reduced sedimentation effect.

In one embodiment, when the method for culturing lactic acid bacteria of the present disclosure comprises the seed culturing step, inoculating the lactic acid bacteria in the culturing step may mean inoculating the seed culture broth obtained in the seed culturing step.

In the culturing step, the temperature of the medium may be 20°C to 50°C, or specifically 35°C to 40°C, but is not limited thereto.

In the culturing step, the culturing may be continued until a desired amount of lactic acid bacteria fermentation broth is obtained, and may specifically be performed for 1 to 50 hours, but is not limited thereto.

The method for culturing lactic acid bacteria of the present disclosure involves culturing lactic acid bacteria using a medium containing ultra-fine red pepper powder to provide an improved growth environment for lactic acid bacteria during fermentation, thereby allowing the provision of a lactic acid bacteria fermentation broth with higher biomass. In one example of the present disclosure, it was confirmed that the cell count of lactic acid bacteria cultured by the method for culturing lactic acid bacteria of the present disclosure was increased compared to that of lactic acid bacteria cultured by a conventional culturing method.

In one embodiment, in the method for culturing lactic acid bacteria of the present disclosure, the viable cell count (CFU/mL) of lactic acid bacteria after culturing may be increased to at least 70-fold, at least 80-fold, or 90-fold to 100-fold or more, relative to the viable cell count (CFU/mL) of the lactic acid bacteria prior to inoculation.

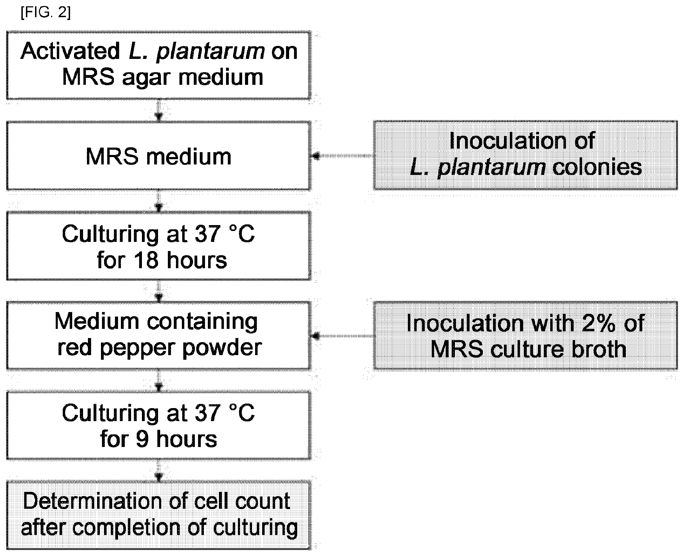

An exemplary method for culturing lactic acid bacteria according to an embodiment of the present disclosure is shown in FIG. 2.

Another aspect of the present disclosure provides a method for increasing the viable cell count of lactic acid bacteria, comprising a culturing step of inoculating and culturing the lactic acid bacteria in a medium composition for culturing lactic acid bacteria comprising ultra-fine red pepper powder as an active ingredient.

The medium composition for culturing lactic acid bacteria and the culturing step are defined in the same manner as described above.

Still another aspect of the present disclosure provides a method for improving the survival rate of lactic acid bacteria, comprising a culturing step of inoculating and culturing the lactic acid bacteria in a medium composition for culturing lactic acid bacteria comprising ultra-fine red pepper powder as an active ingredient.

The medium composition for culturing lactic acid bacteria and the culturing step are defined in the same manner as described above.

Still another aspect of the present disclosure provides a method for reducing sedimentation during fermentation of lactic acid bacteria, comprising a culturing step of inoculating and culturing the lactic acid bacteria in a medium composition for culturing lactic acid bacteria comprising ultra-fine red pepper powder as an active ingredient.

The medium composition for culturing lactic acid bacteria and the culturing step are defined in the same manner as described above.

Still another aspect of the present disclosure provides a lactic acid bacteria fermentation broth, prepared by culturing in the culture medium for culturing lactic acid bacteria or by the method for culturing lactic acid bacteria. In particular, the medium composition for culturing lactic acid bacteria and the method for culturing lactic acid bacteria are defined in the same manner as described above. The lactic acid bacteria fermentation broth of the present disclosure is characterized by increasing the viable cell count of lactic acid bacteria and minimizing the occurrence of sedimentation through fermentation using ultra-fine red pepper powder.

The lactic acid bacteria fermentation broth of the present disclosure may have an improved survival rate compared to that of an existing lactic acid bacteria fermentation broth.

In one embodiment, the lactic acid bacteria fermentation broth of the present disclosure may be characterized by having a viable cell count of lactic acid bacteria of at least 1.9 x 10⁹ CFU/mL, based on an undiluted state. In a more specific embodiment, the lactic acid bacteria fermentation broth may be characterized by having a high concentration of a viable cell count of lactic acid bacteria of 2.0 x 10⁹ CFU/mL to 3.0 x 10⁹ CFU/mL. The above value may be measured at the completion of culturing, *i.e.*, based on a state before the fermentation broth is diluted, and it should be understood that the viable cell count may change when the fermentation broth is diluted for commercialization.

The lactic acid bacteria fermentation broth of the present disclosure may exhibit reduced sedimentation at completion of culturing. The lactic acid bacteria fermentation broth of the present disclosure thus has high applicability in the production of liquid beverages by minimizing the occurrence of sedimentation.

Still another aspect of the present disclosure provides a method for preparing a lactic acid bacteria fermentation broth, comprising inoculating and culturing the lactic acid bacteria in the medium composition for culturing lactic acid bacteria.

In particular, the medium composition for culturing lactic acid bacteria, the lactic acid bacteria, and the lactic acid bacteria fermentation broth are defined in the same manner as described above.

Still another aspect of the present disclosure provides a probiotic beverage composition, comprising the lactic acid bacteria fermentation broth as an active ingredient.

The probiotic beverage composition of the present disclosure is characterized by comprising a high concentration of a viable cell count of the lactic acid bacteria while exhibiting reduced sedimentation, thereby having improved drinkability and stability. Conventional probiotic beverage compositions have limitations of poor drinkability and low storage stability caused by sedimentation in microorganism fermentation broths. The probiotic beverage composition of the present disclosure has a high viable cell count of lactic acid bacteria while exhibiting minimized sedimentation through fermentation using ultra-fine red pepper powder, thereby allowing the provision of a beverage composition with improvements in both drinkability and stability.

In particular, the lactic acid bacteria fermentation broth is defined in the same manner as described above.

As used herein, the term "probiotics" refers to a concept collectively denoting live microorganisms that exert beneficial effects on health, and is used interchangeably with lactic acid bacteria. That is, the probiotic beverage composition of the present disclosure may refer to a beverage comprising lactic acid bacteria that exert beneficial effects on health, such as improvement of intestinal function and enhancement of immunity.

In the probiotic beverage composition, the lactic acid bacteria fermentation broth may be comprised in an amount of 0.01 wt% to 100 wt%, more specifically 1 wt% to 100 wt%; however, the fermentation broth may be provided as-is as a beverage composition, and the amount added may be adjusted according to the intended purpose.

The probiotic beverage composition of the present disclosure may be further added with various known additives conventionally used in the art for preparing probiotic beverages. Examples of the additives include vitamins, trace elements, flavoring agents, flavor substances, and preservatives, but are not limited thereto.

Examples of the vitamins include various water-soluble and fat-soluble vitamins, such as vitamin A (retinols), vitamin B1 (thiamine), vitamin B2 (riboflavin), vitamin B6 (pyridoxine), vitamin B12 (cyanocobalamin), vitamin D (*e.g.*, cholecalciferol), vitamin E (tocopherol), niacin, bisbentiamine, nicotinamide, calcium pantothenate, folic acid, biotin, and choline bitartrate, but are not limited thereto.

Examples of the minerals (electrolytes and trace elements) include manganese sulfate, copper sulfate, zinc sulfate, sodium iodide, potassium sorbate, zinc, manganese, copper, iodine, and cobalt.

Examples of the flavoring agents include apple flavor, orange flavor, grapefruit flavor, lemon flavor, cranberry flavor, lemon flavor, and pineapple flavor.

Examples of the flavor substances include chocolate.

Examples of the preservatives include benzoic acid, sodium benzoate, potassium benzoate, calcium benzoate, methyl parahydroxybenzoate, ethyl parahydroxybenzoate, sorbic acid, potassium sorbate, and calcium sorbate.

Still another aspect of the present disclosure provides a method for preparing a probiotic beverage composition, comprising: a step of preparing a lactic acid bacteria fermentation broth comprising inoculating and culturing lactic acid bacteria in the medium composition for culturing lactic acid bacteria; and a mixing step of mixing the lactic acid bacteria fermentation broth with beverage syrup.

In particular, the medium composition for culturing lactic acid bacteria, the lactic acid bacteria, and the lactic acid bacteria fermentation broth are defined in the same manner as described above.

The beverage syrup may comprise additives such as vitamins, trace elements, flavoring agents, flavor substances, and preservatives, and the additives are defined in the same manner as described above.

In the mixing step, the lactic acid bacteria fermentation broth may be comprised in an amount of 0.01 wt% to 100 wt%, more specifically 1 wt% to 100 wt%, based on the total beverage composition, but is not limited thereto.

In one embodiment, the method for preparing a probiotic beverage composition of the present disclosure may further comprise at least one of a sterilization step, a cooling step, and a homogenization step.

In particular, in the method for preparing lactic acid bacteria of the present disclosure, at least one of the sterilization step, the cooling step, and the homogenization step may each be independently performed once or, if necessary, repeatedly multiple times.

In one embodiment, the probiotic beverage composition of the present disclosure may further comprise a packaging step of filling and packaging the mixture obtained in the mixing step to produce a product.

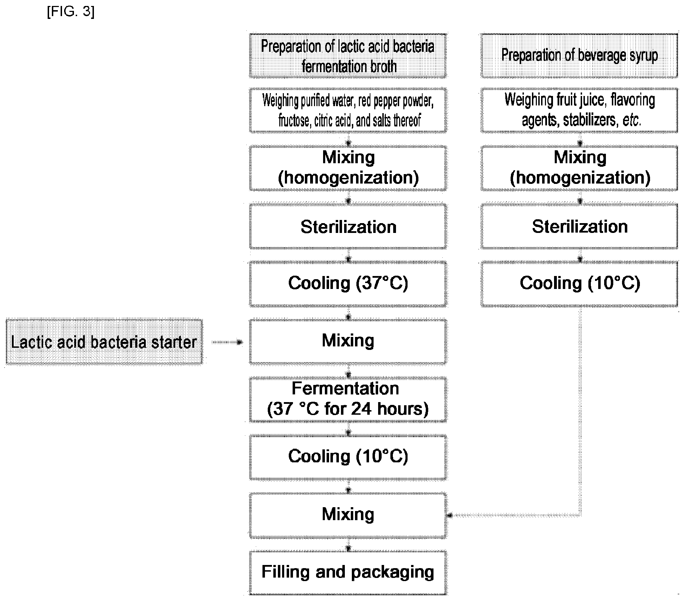

A specific method for preparing the probiotic beverage composition according to an embodiment of the present disclosure is shown in FIG. 3.

Still another aspect of the present disclosure provides a probiotic food composition, comprising the lactic acid bacteria fermentation broth as an active ingredient.

In particular, the lactic acid bacteria fermentation broth and the probiotics are defined in the same manner as described above.

When the lactic acid bacteria fermentation broth of the present disclosure is added to food, it may be added as-is or appropriately used together with other foods or food ingredients according to conventional methods.

In the probiotic food composition, the lactic acid bacteria fermentation broth may be comprised in an amount of 0.01 wt% to 75 wt%; however, the amount added may be adjusted according to the intended purpose.

The formulation of the food may be prepared without limitation, as long as it is a formulation recognized as food. Examples include meat, sausage, bread, chocolate, candies, snacks, confectionery, dairy products, including ice cream, various soups, drinks, and vitamin complexes.

In one embodiment, the food may be a health functional food.

As used herein, the term "health functional food" refers to a food manufactured and processed using raw materials or ingredients having functionality beneficial to the human body in accordance with the Act on Health Functional Foods (Act No. 6727), and the term "functionality" means obtaining effects useful for health purposes, such as regulating nutrients or exerting physiological actions on the structure and functions of the human body. Meanwhile, health food refers to food that has active effects on maintaining or promoting health compared to general foods, and health supplement food refers to food for the purpose of health supplementation. In some cases, the terms "health functional food", "health food", and "health supplement food" may be used interchangeably.

The food of the present disclosure can be prepared by methods commonly used in the art, and during the preparation thereof, raw materials and ingredients commonly added in the art may be added. Specifically, the food composition may further comprise a physiologically acceptable carrier. The type of the carrier is not particularly limited, and any carrier commonly used in the art may be used. In addition, the food composition may include food additives such as preservatives, sterilizers, antioxidants, colorants, color fixatives, bleaching agents, seasonings, sweeteners, flavoring agents, leavening agents, fortifying agents, emulsifiers, thickeners, coating agents, anti-caking agents, anti-foaming agents, solvents, and improvers. The additives may be selected according to the type of food and used in appropriate amounts.

Another aspect of the present disclosure provides use of a medium composition for culturing lactic acid bacteria, the medium composition comprising ultra-fine red pepper powder having an average particle diameter of 10 µm to 50 µm as an active ingredient.

In particular, the medium composition for culturing lactic acid bacteria and the lactic acid bacteria are defined in the same manner as described above.

### [Mode for Carrying Out the Invention]

Hereinafter, the present disclosure is described in detail by way of Examples. The Examples are provided to illustrate the present disclosure in more detail, and the scope of the present disclosure is not limited by the Examples.

### Example 1: Preparation of ultra-fine red pepper powder

For use in the experiments of the present disclosure, commercially available general red pepper powder having a particle size of 12 mesh (1680 µm) was used as a raw material of Comparative Example 1. The 12-mesh red pepper powder was pulverized using a dry air-jet mill method and used as a raw material for the medium of Experimental Example 1.

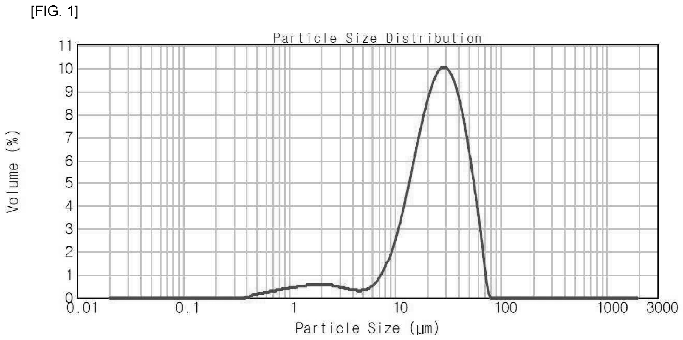

The results of particle size analysis of the ultra-fine red pepper powder are shown in Table 1 below, and the particle size distribution measured using a particle size analyzer is shown in FIG. 1. As can be confirmed from Table 1 and FIG. 1, the ultra-fine red pepper powder had an average particle size of 27.395 µm, a particle size corresponding to 10% (D10) of 9.296 µm, a particle size corresponding to 50% (D50) (*i.e.,* the median particle size) of 25.255 µm, and a particle size corresponding to 90% (D90) of 49.419 µm.

**[Table 1]**

| **Size (um)** | **Vol Under %** | **Size (um)** | **Vol Under %** | **Size (um)** | **Vol Under %** |
|---|---|---|---|---|---|
| 0.105 | 0.00 | 1.096 | 1.75 | 11.482 | 13.81 |
| 0.120 | 0.00 | 1.259 | 2.18 | 13.183 | 17.56 |
| 0.138 | 0.00 | 1.445 | 2.64 | 15.136 | 22.47 |
| 0.158 | 0.00 | 1.660 | 3.13 | 17.378 | 28.54 |
| 0.182 | 0.00 | 1.905 | 3.64 | 19.953 | 35.74 |
| 0.209 | 0.00 | 2.188 | 4.15 | 22.909 | 43.86 |
| 0.240 | 0.00 | 2.512 | 4.65 | 26.303 | 52.63 |
| 0.275 | 0.00 | 2.884 | 5.11 | 30.200 | 61.69 |
| 0.316 | 0.00 | 3.311 | 5.53 | 34.674 | 70.62 |
| 0.363 | 0.00 | 3.802 | 5.88 | 39.811 | 79.00 |
| 0.417 | 0.00 | 4.365 | 6.19 | 45.709 | 86.39 |
| 0.479 | 0.11 | 5.012 | 6.47 | 52.481 | 92.45 |
| 0.550 | 0.26 | 5.754 | 6.79 | 60.256 | 96.85 |
| 0.631 | 0.47 | 6.607 | 7.26 | 69.183 | 99.48 |
| 0.724 | 0.72 | 7.586 | 8.00 | 79.433 | 100.00 |
| 0.832 | 1.02 | 8.710 | 9.21 | 91.201 | 100.00 |
| 0.955 | 1.36 | 10.000 | 11.08 | 104.713 | 100.00 |

### Example 2: Preparation of lactic acid bacteria culture medium

Prior to performing experiments to confirm the effect of the lactic acid bacteria culture medium comprising the ultra-fine red pepper powder of the present disclosure on the promotion of culturing lactic acid bacteria, a medium usable therefor was prepared. A medium of Comparative Example 1 was prepared using common red pepper powder, and a medium of Experimental Example 1 was prepared using the ultra-fine red pepper powder of Example 1. The components of each medium are shown in Table 2 below.

**[Table 2]**

| **Ingredients** | **Comparative Example 1** | **Experimental Example 1** |
|---|---|---|
| Red pepper powder | General red pepper powder (Particle size: 1,680 um) 8 g | Ultra-fine red pepper powder (Average particle size: 27.395 um) 8 g |
| Fructose | 2 g | 2 g |
| Citric acid | 0.8 g | 0.8 g |
| Trisodium citrate | 2.4 g | 2.4 g |
| Water | 85.8 g | 85.8 g |

Red pepper powder and fructose were used as nutrient sources of the medium for culturing lactic acid bacteria, and citric acid and trisodium citrate were comprised as buffers to prevent a rapid decrease in pH caused by lactic acid bacteria fermentation.

Specifically, the culture medium of Comparative Example 1 was prepared by mixing 8 g of general red pepper powder commonly used in foods (average particle size: 1,500 µm), 2 g of fructose, 0.8 g of citric acid, and 2.4 g of trisodium citrate, followed by adding 85.8 g of distilled water and sterilizing the mixture.

Next, the culture medium of Experimental Example 1 was prepared in the same manner as the culture medium of Comparative Example 1, except that only the particle size of the red pepper powder was changed. Specifically, 8 g of ultra-fine red pepper powder prepared by applying an ultra-fine pulverization process (average particle size: 27 µm), 2 g of fructose, 0.8 g of citric acid, and 2.4 g of trisodium citrate were mixed, followed by adding 85.8 g of distilled water and sterilizing the mixture.

### Example 3: Difference in growth effect of lactic acid bacteria according to particle size of red pepper powder and evaluation of culture broth

### Example 3-1: Lactic acid bacteria seed culture

*Lactobacillus plantarum* CJLP133, CJLP243, and CJLP55 colonies activated on MRS solid medium plates were each cultured in MRS liquid medium (Difco, USA) at 37°C for 18 hours. The lactic acid bacteria in the culture broth were confirmed to have a cell count of about 0.91×10⁹ CFU/mL to 1.16×10⁹ CFU/mL.

Specifically, the components and preparation method of the MRS medium are as follows. Proteose peptone 10 g, beef extract 10 g, yeast extract 5 g, dextrose 20 g, polysorbate (Polysorbate 80) 1 g, ammonium citrate 2 g, sodium acetate 5 g, magnesium sulfate 0.1 g, manganese sulfate 0.05 g, and dipotassium phosphate 2 g were mixed, made up to 1 L with water, dissolved, and sterilized.

### Example 3-2: Culturing of lactic acid bacteria in medium containing red pepper powder and growth evaluation

Each of MRS seed cultures containing lactic acid bacteria CJLP133, CJLP243, and CJLP55 was inoculated at 2% into a lactic acid bacteria culture medium containing red pepper powder and cultured at 37°C for 9 hours. By culturing the lactic acid bacteria in the culture media of Comparative Example 1 and Experimental Example 1 prepared above, the growth of the lactic acid bacteria was measured to evaluate the differences in lactic acid bacteria growth between the respective culture media.

A schematic diagram of the lactic acid bacteria fermentation broth preparation process, according to one embodiment of the present disclosure, of Examples 3-1 and 3-2 is shown in FIG. 2.

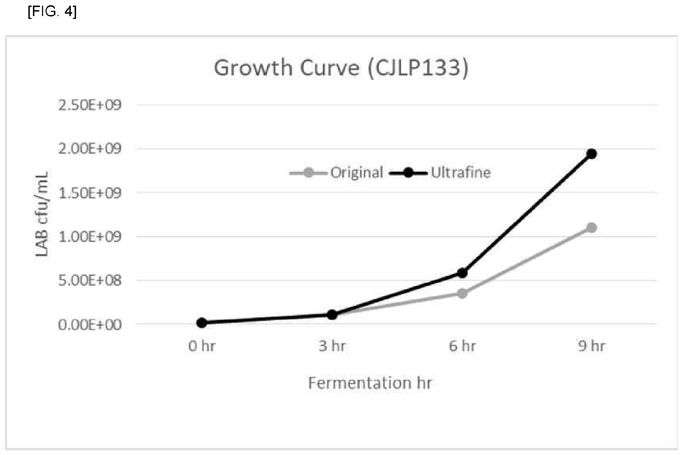

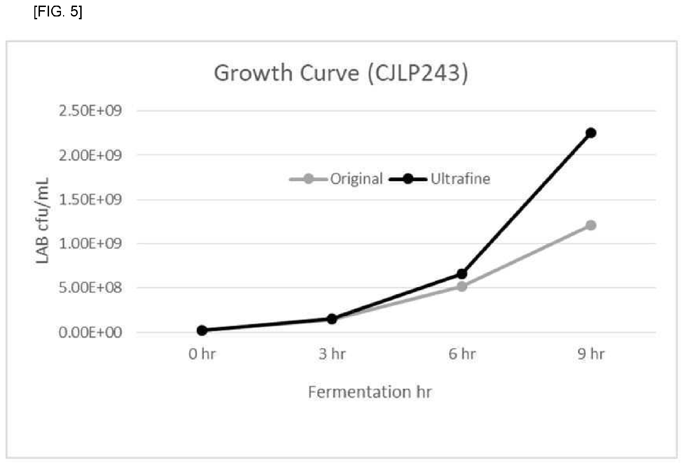

To evaluate the differences in growth, 1 mL of the liquid culture media of Comparative Example 1 and Experimental Example 1 was sampled and diluted (dilution ratio: 10⁵-10⁶), followed by culturing on plate media. Specifically, the culture broths of the lactic acid bacteria strains *Lactobacillus plantarum* CJLP133, CJLP243, and CJLP55 were diluted with sterile saline so as to form about 30 to 300 colonies on plate media, spread thereon, and cultured at 37°C for 24 hours. After 24 hours of culturing, the number of colonies formed by the lactic acid bacteria was counted and calculated as the viable cell count per mL. The results are shown in Tables 3 to 5 below, and growth curves of the media for each strain are shown in FIGS. 4 to 6.

**[Table 3]**

| **CJLP133 (CFU/ml)** | **Comparative Example 1** | **Experimental Example 1** |
|---|---|---|
| | **General red pepper powder** | **Ultra-fine red pepper powder** |
| 0 hours | 1.82 x 10⁷ | 1.82 x 10⁷ |
| 3 hours | 1.15 x 10⁸ | 1.10 x 10⁸ |
| 6 hours | 3.52 x 10⁸ | 5.88 x 10⁸ |
| 9 hours | 1.10 x 10⁹ | 1.95 x 10⁹ |

**[Table 4]**

| **CJLP243 (CFU/mL)** | **Comparative Example 1** | **Experimental Example 1** |
|---|---|---|
| | **General red pepper powder** | **Ultra-fine red pepper powder** |
| 0 hours | 2.32 x 10⁷ | 2.32 x 10⁷ |
| 3 hours | 1.41 x 10⁸ | 1.54 x 10⁸ |
| 6 hours | 5.16 x 10⁸ | 6.58 x 10⁸ |
| 9 hours | 1.21 x 10⁹ | 2.25 x 10⁹ |

**[Table 5]**

| **CJLP55 (CFU/mL)** | **Comparative Example 1** | **Experimental Example 1** |
|---|---|---|
| | **General red pepper powder** | **Ultra-fine red pepper powder** |
| 0 hours | 2.16 x 10⁷ | 2.16 x 10⁷ |
| 3 hours | 1.35 x 10⁸ | 1.23 x 10⁸ |
| 6 hours | 4.26 x 10⁸ | 6.11 x 10⁸ |
| 9 hours | 0.98 x 10⁹ | 2.10 x 10⁹ |

As can be seen from Tables 3 to 5 and FIGS. 4 to 6, in all of the lactic acid bacteria CJLP133, CJLP243, and CJLP55, the viable cell count per mL of the medium of Experimental Example 1 using ultra-fine red pepper powder was confirmed to be significantly higher than that of the medium of Comparative Example 1 using general red pepper powder. These results suggest that the medium of the present disclosure, comprising ultra-fine red pepper powder, exhibits superior effects in increasing the growth and viable cell count of lactic acid bacteria compared to conventional media.

### Example 3-3: Evaluation of beverage suitability of fermentation culture broth according to particle size of red pepper powder

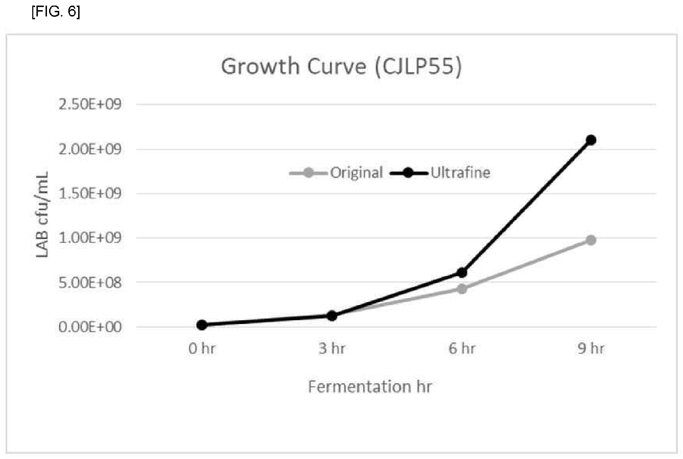

The results confirmed after completion of the culturing performed through Examples 3-1 and 3-2 are shown in FIG. 6 below.

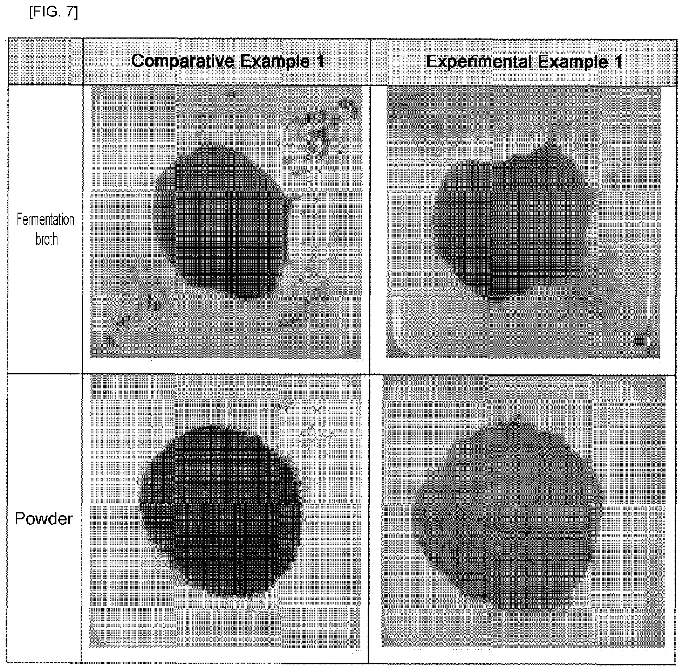

As can be seen in FIG. 7, a large amount of red pepper powder was sedimented at the bottom of the fermentation broth cultured in the medium of Comparative Example 1 using general red pepper powder, whereas in the fermentation broth cultured in the medium of Experimental Example 1 containing ultra-fine red pepper powder, no sediment or layer separation was observed. From these results, it was confirmed that the medium of the present disclosure and the fermentation broth produced using the same exhibit minimized sedimentation during fermentation, thereby exhibiting high applicability for use in food production, such as beverages.

As set forth above, those skilled in the art will be able to understand that the present disclosure may be embodied in other specific forms without departing from the technical spirit or essential characteristics thereof. In this regard, it should be understood that the foregoing examples are illustrative in all respects and are not to be construed as limiting. The scope of the present disclosure should be construed as including the meaning and scope of the appended claims rather than the detailed description, and all changes or variations derived from the equivalent concepts fall within the scope of the present disclosure.

This work was supported by the Korea Institute of Planning and Evaluation for Technology in Food, Agriculture and Forestry, funded by the Ministry of Agriculture, Food and Rural Affairs (High Value-added Food Technology Development Program) (121012-3).

## Claims

1. A medium composition for culturing lactic acid bacteria, comprising ultra-fine red pepper powder having an average particle size of 10 µm to 50 µm as an active ingredient.

2. The medium composition according to claim 1, wherein the ultra-fine red pepper powder has a particle size distribution in which D10, D50, and D90 are all 70 µm or less.

3. The medium composition according to claim 1, wherein the ultra-fine red pepper powder has a particle size distribution in which D10 is 1 µm to 15 µm, D50 is 10 µm to 35 µm, and D90 is 30 µm to 70 µm.

4. The medium composition according to claim 1, wherein the average particle size of the ultra-fine red pepper powder is 20 µm to 30 µm.

5. The medium composition according to claim 1, wherein the ultra-fine red pepper powder is comprised in an amount of 1 wt% to 20 wt% based on the total weight of the medium composition.

6. The medium composition according to claim 1, further comprising: fructose; a buffer solution comprising at least one of citric acid and sodium citrate; and a remaining amount of purified water.

7. The medium composition according to claim 1, wherein the lactic acid bacteria comprise at least one selected from the group consisting of *Lactobacillus* sp., *Bifidobacterium* sp., *Streptococcus* sp., *Lactococcus* sp., *Enterococcus* sp., *Pediococcus* sp., *Leuconostoc* sp., and *Weissella* sp.

8. The medium composition according to claim 1, wherein the lactic acid bacteria comprise at least one selected from the group consisting of *Lactobacillus plantarum* CJLP133, *Lactobacillus plantarum* CJLP243, and *Lactobacillus plantarum* CJLP55.

9. A method for culturing lactic acid bacteria, comprising a culturing step of inoculating and culturing the lactic acid bacteria in the medium composition for culturing lactic acid bacteria according to any one of claims 1 to 8.

10. The method according to claim 9, further comprising a seed culturing step of obtaining a seed culture broth by culturing the lactic acid bacteria in a seed medium before the culturing step,
wherein the inoculation of the lactic acid bacteria is inoculating the seed culture broth obtained in the seed culturing step.

11. The method according to claim 9, wherein the viable cell count (CFU/mL) of the lactic acid bacteria after culturing is increased to at least 90 times the viable cell count (CFU/mL) of the lactic acid bacteria before inoculation.

12. A method for increasing the viable cell count of lactic acid bacteria, comprising a culturing step of inoculating and culturing the lactic acid bacteria in the medium composition for culturing lactic acid bacteria according to any one of claims 1 to 8.

13. A lactic acid bacteria fermentation broth, prepared by the method for culturing lactic acid bacteria according to claim 9.

14. The lactic acid bacteria fermentation broth according to claim 13, having an improved survival rate of the lactic acid bacteria and reduced sedimentation.

15. The lactic acid bacteria fermentation broth according to claim 13, having a high concentration of a viable cell count of the lactic acid bacteria of 2.0 x 10⁹ CFU/mL to 3.0 x 10⁹ CFU/mL.

16. A probiotic beverage composition, comprising the lactic acid bacteria fermentation broth of claim 13 as an active ingredient.

17. The probiotic beverage composition of claim 16, comprising a high concentration of a viable cell count of the lactic acid bacteria and having improved drinkability and stability.

18. A probiotic food composition, comprising the lactic acid bacteria fermentation broth of claim 13 as an active ingredient.
